# FASCICULE DE BREVET EUROPEEN

(11) **EP 2 965 720 B1**
(45) Date de publication et mention de la délivrance du brevet: **05.07.2017**
(21) Numéro de dépôt: 15175958.6
(22) Date de dépôt: 08.07.2015
(51) Int. Cl.: A61F 2/40, A61B 17/86, A61F 2/30, A61F 2/46

(54) **EMBASE D'ANCRAGE POUR IMPLANT ORTHOPÉDIQUE D'ARTICULATION**
VERANKERUNGSPLATTE FÜR ORTHOPÄDISCHES GELENKIMPLANTAT
ANCHORING BASE FOR ORTHOPAEDIC JOINT IMPLANT

(30) Priorité: 10.07.2014 FR 1456685
(43) Date de publication de la demande: 13.01.2016
(73) Titulaire: Evolutis, 42720 Briennon (FR)
(72) Inventeur: OUDET, Didier, 37540 SAINT CYR SUR LOIRE (FR); LE DU, Christophe, 37510 BALLAN MIRE (FR); MARTIN, Jean-Noël, 76130 Mont Saint Aignan (FR); CHAUMEIL, Géraud, 81000 ALBI (FR); GOSSELIN, Olivier, 54690 LAY SAINT CHRISTOPHE (FR); CUISINIER, Alan-Bernard, 76000 ROUEN (FR); BEGUIN, Laurent, 69390 CHARLY (FR)
(74) Mandataire: Cabinet Laurent & Charras

(56) Documents cités:
- WO-A1-03/051238
- WO-A1-2014/005644
- US-A1- 2007 142 917
- US-A1- 2013 261 629
- US-A1- 2014 121 709

## Description

L'invention se rattache au secteur technique des implants orthopédiques.

L'invention trouve une application particulièrement avantageuse dans le cas d'une prothèse d'articulation d'épaule.

De manière parfaitement connue pour un homme du métier et de manière générale, ce type de prothèse comprend un implant huméral fixé dans la diaphyse médullaire de l'humérus et apte à coopérer avec un implant glénoïdien fixé au niveau de la glène anatomique de l'omoplate. Généralement, l'implant huméral présente une tête d'articulation hémisphérique assujettie à une tige d'ancrage engagée dans la diaphyse humérale. L'implant glénoïdien présente une cupule ou platine fixée, comme indiqué, au niveau de la glène anatomique.

On a proposé également, notamment pour déplacer vers l'omoplate le centre de rotation et accroitre l'efficacité du muscle deltoïde, d'inverser ce principe d'articulation. Autrement dit, dans ce cas de prothèse dite inversée, la tête d'articulation en tant que telle est fixée au niveau de la glène pour coopérer avec une cupule de forme complémentaire fixée au niveau de l'humérus.

Quel que soit le type de prothèse choisie, dans la plupart des cas, au niveau de l'implant huméral, ce dernier est fixé dans la diaphyse de l'humérus au moyen d'une tige engagée dans ladite diaphyse. La mise en place de cette tige humérale nécessite d'utiliser une râpe susceptible d'imposer un angle cervico-diaphysaire résultant de la coupe de la tête anatomique humérale.

On a proposé, par exemple, dans le cadre d'une prothèse d'articulation inversée, de ne plus utiliser de tige humérale. Une telle solution ressort de la demande de brevet FR 12 60204.

Dans le domaine de l'arthroplastie de l'articulation de l'épaule, notamment dans le cas d'une omarthrose centrée primitive, le praticien peut, soit utiliser une prothèse totale anatomique autrement dit utiliser un implant huméral avec une tige impactée dans la diaphyse de l'humérus, soit utiliser un simple resurfaçage au moyen d'une cupule fine emboitée sur la tête humérale.

Différentes solutions ont été proposées pour réaliser des prothèses d'épaule dites sans-tige comme il ressort par exemple de l'enseignement des documents EP 2 502 606, WO 2013/103499.

Le document WO 2014/005644, correspondant au préambule de la revendication 1, propose pour sa part une embase comprenant une couronne, un plot et des ailettes d'ancrage formées en dessous de la couronne.

A partir de cet état de la technique, le problème que se propose de résoudre l'invention est de pouvoir réaliser un implant orthopédique qui soit moins invasif dans le patient, qui permette de préserver le capital osseux, autrement dit, ne pas utiliser une tige d'ancrage remplissant en grande partie le canal médullaire de l'os considéré, de pouvoir respecter l'anatomie et de permettre une bonne repousse osseuse et une bonne stabilité immédiate et dans le temps. Et dernier point: malgré la bonne tenue dans l'os de l'implant, ce dernier doit pouvoir être retiré par le chirurgien en cas de nécessité, par exemple en cas d'infection.

Pour résoudre ce problème, il a été conçu et mis au point une embase d'ancrage pour implant orthopédique d'articulation, comprenant une surface d'appui plane sous forme d'une couronne apte à coopérer avec une coupe osseuse réalisée à l'extrémité métaphysaire de l'os considéré, ladite couronne présentant coaxialement un plot assujetti à des ailettes d'ancrage aptes à être impactées dans la coupe osseuse. Selon l'invention, les ailettes d'ancrage sont formées radialement à partir de la périphérie de la couronne et ajourées par l'adjonction d'une paroi grillagée.

Il résulte de ces caractéristiques que la couronne permet d'obtenir une parfaite stabilité évitant tout phénomène d'enfoncement sous certains efforts, tout en permettant une repousse osseuse importante et une parfaite fixation, avec la possibilité d'extraction de cette embase au moyen notamment d'agencement spécifique que peut présenter le plot.

Les parois grillagées sont très fines, de manière à favoriser la repousse osseuse. Selon l'invention, ces parois ont une épaisseur de l'ordre de 0,1 à 0,3 mm, correspondant sensiblement aux cellules osseuses.

Les espaces formés entre la couronne et le plot permettent le passage d'outils tel qu'un ciseau afin de couper les ponts osseux formés lors de la repousse osseuse dans les zones ajourées des ailettes afin de rendent possible l'extraction de l'embase. Selon l'invention, l'embase peut être réalisée par fabrication additive, en mettant en oeuvre un dispositif d'impression en trois dimensions. Ce procédé est bien adapté, entre autres avantages, pour obtenir des parois grillagées d'une grande finesse.

Pour résoudre le problème posé d'obtenir une parfaite stabilité tout en permettant une parfaite repousse osseuse, chaque ailette est constituée par un segment rectiligne qui relie le plot et la couronne et par un segment profilé qui relie, au niveau du raccordement dudit segment rectiligne, ladite couronne avec le plot. La paroi grillagée s'étend entre le plot, le segment rectiligne et le segment profilé.

Avantageusement, les ailettes sont décalées angulairement sur la circonférence de la couronne. Par exemple, les ailettes sont au nombre de quatre en étant décalées de 90°.

Selon une autre caractéristique, le plot présente des agencements pour l'accouplement de l'implant orthopédique, notamment sous forme d'un cône morse. Le cône morse est soit male, soit femelle. Le plot est creux.

Un autre problème dans le contexte de l'invention est d'améliorer la fixation de l'embase d'ancrage. Dans ce but, l'extrémité du plot considéré à l'opposé de la couronne peut avantageusement être taraudée pour coopérer avec une vis à corticale.

L'invention s'applique à tout type d'implant orthopédique d'articulation et trouve une application particulièrement avantageuse dans le cas d'une prothèse d'articulation de l'épaule.

Dans ce but, l'implant orthopédique est constitué par une cupule hémisphérique mâle pour faire office de tête humérale apte à coopérer avec un implant glénoïdien sous forme d'une cupule hémisphérique femelle.

Dans le cas d'une prothèse d'articulation d'épaule dite inversée, l'implant orthopédique est constitué par une cupule hémisphérique femelle pour faire office de cupule humérale apte à coopérer avec un implant glénoïdien sous forme d'une cupule hémisphérique mâle.

L'invention est exposée ci-après plus en détail à l'aide des figures des dessins annexés dans lesquels :
- la figure 1 est une vue en perspective de l'embase d'ancrage considérée par le dessus ;
- la figure 2 est une vue en perspective de l'embase d'ancrage considérée par le dessous ;
- la figure 3 montre la mise en place et l'impaction de l'embase d'ancrage dans le cas d'un implant huméral anatomique.

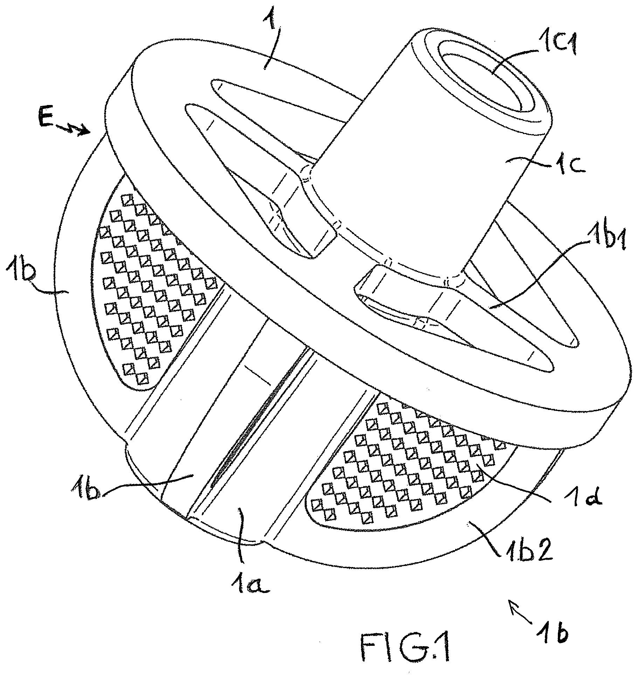

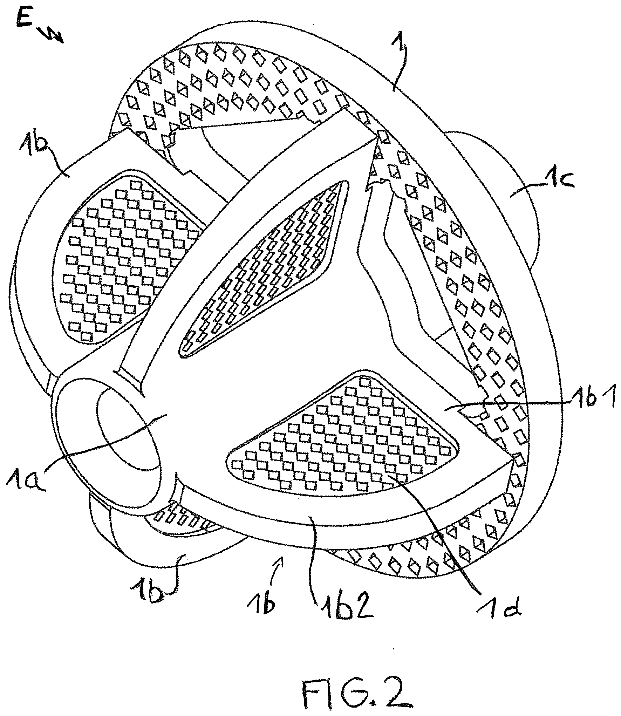

Comme le montrent les figures 1 et 2, l'embase de fixation désignée dans son ensemble par (E) comprend une surface d'appui plane sous forme d'une couronne (1). Comme il sera indiqué dans la suite de la description, cette couronne (1) est apte à coopérer avec une coupe osseuse réalisée à l'extrémité métaphysaire de l'os considéré, par exemple, un humérus ou un fémur.

Cette couronne (1) présente co-axialement un plot (1a) assujetti à des ailettes d'ancrage (1b) formées radialement à partir de la périphérie de ladite couronne (1). Le plot (1a) et les ailettes (1b) apparaissent en débordement de la face de dessous d'appui de la couronne (1) pour être impactés dans la coupe osseuse.

Pour faciliter la repousse osseuse, les ailettes (1b) sont ajourées et avantageusement remplies par un grillage ajouré, formant une paroi grillagée (1d). Plus particulièrement, chaque ailette (1b) est constituée par un segment rectiligne (1b1) qui relie le plot (1a) à la périphérie de la couronne et par un segment profilé (1b2) sensiblement en arc de cercle qui relie, au niveau du raccordement du segment (1b1) avec la couronne (1), ladite couronne (1) avec le plot (1a). La paroi grillagée (1d) s'étend entre le plot (1b), le segment rectiligne (1b1) et le segment profilé (1b2). Les ailettes (1b) sont décalées angulairement sur la circonférence de la couronne (1). Par exemple, sur les figures des dessins la couronne (1) présente quatre ailettes décalées angulairement de 90°. Les ailettes (1b) sont aptes à être impactées avec le plot (1a) dans la coupe osseuse

Il apparait donc que la couronne (1) et les ailettes (1b) permettent un ancrage sûr et efficace de l'embase (E) sans risque de basculement en considérant la surface d'appui générée par la couronne (1) tout en permettant une parfaite repousse osseuse en considérant les espaces vides que présentent à la fois la couronne et les ailettes ajourées (1b).

Le plot (1a) présente, au niveau de la couronne (1), des agencements pour l'accouplement de différents implants orthopédiques en fonction du type de prothèse d'articulation souhaité. Aux figures des dessins, le plot (1a) est prolongé en débordement de la couronne (1) par une portée tronconique (1c) faisant office de cône morse male. Sur cette portée tronconique (1c) peut être emmanché en force l'implant d'articulation dont la forme de réalisation dépend du type de prothèse d'articulation, comme indiqué.

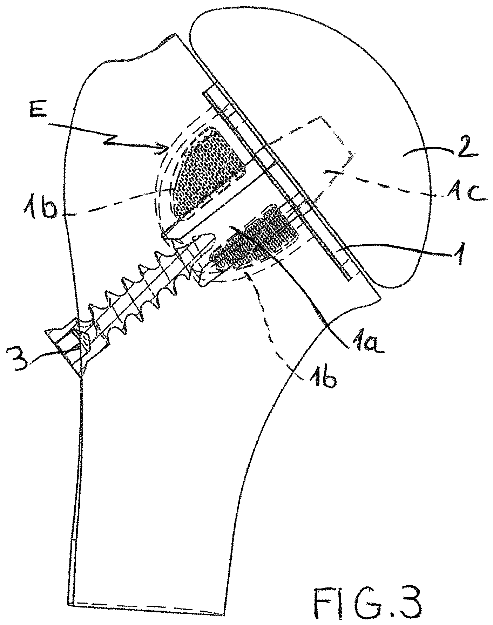

Par exemple, dans le cas d'une prothèse d'articulation de l'épaule anatomique, l'implant orthopédique (2) est constitué par une cupule hémisphérique mâle faisant office de tête humérale, figure 3. Cette tête humérale prothétique est apte à coopérer de manière parfaitement connue avec un implant glénoïdien sous forme d'une cupule hémisphérique femelle (non représentée) ou bien, dans le cas d'une prothèse d'épaule inversée, l'implant orthopédique (2) est constitué par une cupule femelle apte à coopérer avec une cupule hémisphérique mâle constituant la sphère d'articulation en tant que telle fixée au niveau de la cavité anatomique glénoïdienne. Dans ce cas la portée conique sera avantageusement femelle.

Dans une autre forme de réalisation pour l'accouplement de l'implant orthopédique, le plot (1a), au niveau de son raccordement avec la couronne (1), peut présenter une empreinte en creux avec des formes d'indexation aptes à recevoir une portée mâle de section complémentaire que présente directement l'implant orthopédique ou que présente une platine support recevant ledit implant orthopédique. Dans ce cas, la platine support recevant l'implant orthopédique peut être visée dans un trou taraudé formé dans le fond de l'empreinte en creux que présente le plot (1a).

Selon une autre caractéristique, pour améliorer la fixation de l'embase d'ancrage (E), l'extrémité du plot (1a) considérée à l'opposé de la couronne est taraudée pour coopérer avec une vis à corticale (3), figure 3.

Pour permettre, si nécessaire, l'extraction de l'ensemble de l'embase d'ancrage (1), le plot (1) par exemple, le cône morse (1c), peut présenter un taraudage (1c1) pour la mise en place d'un outil d'extraction.

L'impaction de l'embase d'ancrage (E) au niveau de la coupe osseuse est réalisée par tout moyen connu et approprié après avoir préalablement préparée cette coupe osseuse notamment en réalisant les empreintes nécessaires pour l'engagement du plot (1a) et des ailettes (1b).

A noter que la couronne (1) peut prendre simplement appui sur la coupe osseuse réalisée ou être encastrée dans cette dernière. Le plot (1a) et les ailettes (1b) peuvent, par ailleurs, présenter tout type d'aspérités ou de traitement externe apte à améliorer l'ancrage dans la partie de l'os considéré.

Les avantages ressortent bien de la description, en particulier, on rappelle et on souligne :
- la parfaite stabilité de la couronne d'appui et une parfaite repousse osseuse résultant de la couronne et des ailettes creuses,
- la repousse osseuse favorisée par la finesse des parois grillagées formées sur les ailettes,
- la suppression d'une tige d'ancrage dans la partie du canal médullaire de l'os considéré,
- la possibilité de combiner l'embase d'ancrage avec une vis à corticale coaxialement opposée à la fixation de l'implant orthopédique.
- la facilité d'extraction grâce à la couronne qui permet le passage d'outil pour libérer les ailettes du contact osseux.

## Revendications

1. Embase d'ancrage (E) pour implant orthopédique d'articulation, comprenant une surface d'appui plane sous forme d'une couronne (1) apte à coopérer avec une coupe osseuse réalisée à l'extrémité métaphysaire de l'os considéré, ladite couronne (1) présentant coaxialement un plot (1a) assujetti à des ailettes d'ancrage (1b) aptes à être impactées dans la coupe osseuse, les ailettes d'ancrage (1b) étant formées radialement à partir de la périphérie de la couronne (1) **caractérisée en ce que** les ailettes d'ancrage (1b) sont ajourées par l'adjonction d'une paroi grillagée (1d),très fine d'épaisseur, de l'ordre de 0,1 à 0,3 mm correspondant sensiblement aux cellules osseuses afin de favoriser la repousse osseuse, l'embase, y compris les parois grillagées, étant réalisée par fabrication additive.

2. Embase d'ancrage (E) selon la revendication 1, ***caractérisée* en ce que** chaque ailette (1b) est constituée par un segment rectiligne (1b1) qui relie le plot (1a) et la couronne (1) et par un segment profilé (1b2) qui relie, au niveau du raccordement dudit segment rectiligne, ladite couronne (1) avec le plot (1a).

3. Embase d'ancrage (E) selon l'une quelconque des revendications 1 ou 2, ***caractérisée* en ce que** les ailettes (1b) sont décalées angulairement sur la circonférence de la couronne (1).

4. Embase d'ancrage (E) selon l'une quelconque des revendications 1 à 3, ***caractérisée* en ce que** les ailettes (1b) sont au nombre de quatre en étant décalées de 90°.

5. Embase d'ancrage (E) selon l'une quelconque des revendications 1 à 4, ***caractérisée* en ce que** le plot (1a) présente des agencements pour l'accouplement de l'implant orthopédique, notamment sous forme d'un cône morse.

6. Embase d'ancrage (E) selon l'une quelconque des revendications 1 à 5, ***caractérisée* en ce que** le plot (1a) est creux.

7. Embase d'ancrage (E) selon l'une quelconque des revendications 1 à 6, ***caractérisée* en ce que** l'extrémité du plot (1a) considéré à l'opposé de la couronne (1) est taraudée pour coopérer avec une vis à corticale.

8. Embase d'ancrage (E) selon l'une quelconque des revendications 1 à 7, ***caractérisée* en ce que** la couronne (1), le plot (1a) et les ailettes (1b) présentent des aspérités ou traitements externes aptes à améliorer l'ancrage dans la partie de l'os considéré.

9. Prothèse comprenant une embase d'ancrage (E) selon l'une des revendications 1 à 8 et un implant orthopédique (2) constitué par une cupule hémisphérique mâle, pour faire office de tête humérale apte à coopérer avec un implant glénoïdien sous forme d'une cupule femelle.

10. Prothèse comprenant une embase d'ancrage (E) selon l'une des revendications 1 à 8 et un implant orthopédique (2) constitué par une cupule hémisphérique femelle, pour faire office de cupule humérale apte à coopérer avec un implant glénoïdien sous forme d'une cupule hémisphérique mâle.

## Patentansprüche

1. Verankerungsbasis (E) für ein orthopädisches Gelenkimplantat, welche eine ebene Auflagefläche in Form eines Kranzes (1) umfasst, die dazu geeignet ist, mit einem Knocheneinschnitt zusammenzuwirken, welcher am Metaphysenende des betreffenden Knochens vorgenommen wurde, wobei der Kranz (1) koaxial mit einem Zapfen (1a) versehen ist, auf den Verankerungsflügel (1b) einwirken, welche in den Knocheneinschnitt vorstoßen können, wobei die Verankerungsflügel (1b) ausgehend vom Randbereich des Kranzes (1) in radialer Richtung ausgebildet sind, **dadurch gekennzeichnet, dass** die Verankerungsflügel (1b) derart durchbrochen sind, dass sie mit einer Gitterwand (1d) versehen sind, deren Dicke sehr gering ist, nämlich in der Größenordnung von 0,1 bis 0,3 mm, wobei sie im Wesentlichen den Knochenzellen entspricht, um das Nachwachsen des Knochens zu begünstigen, wobei die Basis einschließlich der Gitterwände mittels additiver Fertigung hergestellt wird.

2. Verankerungsbasis (E) nach Anspruch 1, **dadurch *gekennzeichnet*, dass** jeder der Flügel (1b) aus einem geradlinigen Segment (1b1) besteht, welches den Zapfen (1a) mit dem Kranz (1) verbindet, und aus einem Profilsegment (1b2), welches den Kranz (1), im Bereich des Anschlusses an das geradlinige Segment, mit dem Zapfen (1a) verbindet.

3. Verankerungsbasis (E) nach einem beliebigen der Ansprüche 1 oder 2, **dadurch *gekennzeichnet*, dass** die Flügel (1b) einen Winkelversatz zum Umfang des Kranzes (1) aufweisen.

4. Verankerungsbasis (E) nach einem beliebigen der Ansprüche 1 bis 3, **dadurch *gekennzeichnet*, dass** die Anzahl der Flügel (1b) vier beträgt, wobei diese um 90° versetzt sind.

5. Verankerungsbasis (E) nach einem beliebigen der Ansprüche 1 bis 4, **dadurch *gekennzeichnet,* dass** der Zapfen (1a) Ausgestaltungen zur Kupplung mit dem orthopädischen Implantat aufweist, insbesondere in Form eines Morsekegels.

6. Verankerungsbasis (E) nach einem beliebigen der Ansprüche 1 bis 5, **dadurch *gekennzeichnet*, dass** der Zapfen (1a) hohl ist.

7. Verankerungsbasis (E) nach einem beliebigen der Ansprüche 1 bis 6, **dadurch *gekennzeichnet*, dass** das Ende des betreffenden Zapfens (1a) auf der dem Kranz (1) gegenüberliegenden Seite ein Hohlgewinde aufweist, um mit einer Kortikalschraube zusammenzuwirken.

8. Verankerungsbasis (E) nach einem beliebigen der Ansprüche 1 bis 7, **dadurch *gekennzeichnet,* dass** der Kranz (1), der Zapfen (1a) und die Flügel (1b) Unebenheiten oder äußere Behandlungen aufweisen, die dazu geeignet sind, die Verankerung in dem Abschnitt des betreffenden Knochens zu verbessern.

9. Prothese, die eine Verankerungsbasis (E) nach einem der Ansprüche 1 bis 8 und ein orthopädisches Implantat (2) umfasst, welches aus einem vorgewölben halbkugelartigem Napf besteht, um als Oberarmknochenkopf zu dienen, der zu befähigt ist, mit einem Schultergelenkpfannen-Implantat in Form eines hohlen Napfes zusammenzuwirken.

10. Prothese, die eine Verankerungsbasis (E) nach einem der Ansprüche 1 bis 8 und ein orthopädisches Implantat (2) umfasst, welches aus einem hohlen halbkugelartigem Napf besteht, um als Oberarmknochennapf zu dienen, der zu befähigt ist, mit einem Schultergelenkpfannen-Implantat in Form eines vorgewölbten halbkugelartigen Napfes zusammenzuwirken.

## Claims

1. Anchoring base (E) for an orthopedic joint implant, comprising a flat bearing surface in the form of a crown (1) suitable to cooperate with a bone cut made at the metaphyseal end of the relevant bone, said crown (1) coaxially having a stud (1a) secured to anchoring fins (1b) capable of being impinged in the bone section, the anchoring fins (1b) being formed radially from the periphery of the crown (1), **characterized in that** the anchoring fins (1b) are perforated by the addition of a very thin mesh wall (1d), with a thickness in the range from 0.1 to 0.3 mm corresponding substantially to the bone cells in order to promote bone regrowth, the base, including the mesh walls, being produced by additive manufacturing.

2. Anchoring base (E) according to claim 1, **characterized in that** each fin (1b) is constituted by a rectilinear segment (1b1) which connects the stud (1a) and the crown (1) and by a profiled segment (1b2) which connects said crown (1) to the stud (1a) at the connection of said rectilinear segment,

3. Anchoring base (E) according to anyone of claims 1 or 2, **characterized in that** the fins (1b) are angularly offset on the circumference of the crown (1).

4. Anchoring base (E) according to anyone of claims 1 to 3, **characterized in that** the fins (1b) are four in number, offset by 90°.

5. Anchoring base (E) according to anyone of claims 1 to 4, **characterized in that** the stud (1a) has arrangements for the coupling of the orthopedic implant, in particular in the form of a morse cone.

6. Anchoring base (E) according to anyone of claims 1 to 5, **characterized in that** the stud (1a) is hollow.

7. Anchoring base (E) according to anyone of claims 1 to 6, **characterized in that** the end of the stud (1a) considered opposite to the crown (1) is threaded to cooperate with a cortical screw.

8. Anchoring base (E) according to anyone of claims 1 to 7, **characterized in that** the crown (1), the stud (1a) and the fins (1b) have external roughnesses or treatments capable of improving the anchoring in the part of the relevant bone.

9. Prosthesis comprising an anchorage base (E) according to one of claims 1 to 8 and an orthopedic implant (2) made up of a male hemispherical cup, provided to act as a humeral head able to cooperate with a glenoid implant in the form of a female cup.

10. Prosthesis comprising an anchoring base (E) according to one of claims 1 to 8 and an orthopedic implant (2) constituted by a female hemispherical cup, provided to act as a humeral cup able to cooperate with a glenoid implant in the form of a male hemispherical cup.
